(19) **Europäisches Patentamt / European Patent Office / Office européen des brevets**

(11) **EP 1 545 655 B1**

(12) **EUROPEAN PATENT SPECIFICATION**

(45) Date of publication and mention
of the grant of the patent:
**31.12.2008 Bulletin 2009/01**

(51) Int Cl.:
*A61M 5/00* (2006.01)  *A61M 37/00* (2006.01)
*A61B 3/16* (2006.01)  *A61B 5/00* (2006.01)
*A61F 9/007* (2006.01)  *A61M 27/00* (2006.01)

(21) Application number: **03785064.1**

(22) Date of filing: **07.08.2003**

(86) International application number:
**PCT/US2003/024890**

(87) International publication number:
**WO 2004/014218 (19.02.2004 Gazette 2004/08)**

(54) **IMPLANTABLE OCULAR PUMP TO REDUCE INTRAOCULAR PRESSURE**

IMPLANTIERBARE OKULARE PUMPE ZUR VERRINGERUNG DES AUGENINNENDRUCKS

POMPE OCULAIRE IMPLANTABLE DESTINEE A REDUIRE LA PRESSION INTRAOCULAIRE

(84) Designated Contracting States:
**AT BE BG CH CY CZ DE DK EE ES FI FR GB GR HU IE IT LI LU MC NL PT RO SE SI SK TR**

(30) Priority: **08.08.2002 US 402230 P**

(43) Date of publication of application:
**29.06.2005 Bulletin 2005/26**

(73) Proprietor: **Glaukos Corporation
Laguna Hills, CA 92653 (US)**

(72) Inventors:
• **SMEDLEY, Gregory
Aliso Viejo, CA 92656 (US)**

• **HAFFNER, David
Mission Viejo, CA 92692 (US)**
• **TU, Hosheng
Newport Beach, CA 92657 (US)**
• **GHARIB, Morteza
San Marino, CA 91108 (US)**

(74) Representative: **Hector, Annabel Mary et al
D Young & Co
120 Holborn
London EC1N 2DY (GB)**

(56) References cited:
**WO-A-01/94784**  **US-A- 5 433 701**
**US-B1- 6 168 575**  **US-B1- 6 261 256**
**US-B1- 6 589 198**

**Description**

Field of the Invention

The invention generally relates to medical devices for the reduction of elevated pressure in organs of the human body. More particularly, the invention relates to the treatment of glaucoma by implanting a trabecular pump in an eye to reduce intraocular pressure to a desired level by draining aqueous from the anterior chamber into Schlemm's canal or downstream therefrom.

Background of the Invention

About two percent of people in the United States have glaucoma. Glaucoma is a group of eye diseases that causes pathological changes in the optic disk and corresponding visual field loss, resulting in blindness if untreated. Intraocular pressure elevation is a major etiologic factor in glaucoma.

In glaucomas associated with an elevation in eye pressure the source of resistance to outflow is in the trabecular meshwork. The tissue of the trabecular meshwork allows aqueous humor ("aqueous") to enter Schlemm's canal, which then empties into aqueous collector channels in the posterior wall of Schlemm's canal and then into aqueous veins. The aqueous is a transparent liquid that fills the region between the cornea at the front of the eye and the lens. The aqueous is constantly secreted by the ciliary body around the lens, so there is a continuous flow of aqueous humor from the ciliary body to the eye's anterior (front) chamber. The eye's pressure is determined by a balance between the production of aqueous and its exit through the trabecular meshwork (major route) or via uveal scleral outflow (minor route). The trabecular meshwork is located between the outer rim of the iris and the internal periphery of the cornea. The portion of the trabecular meshwork adjacent to Schlemm's canal causes most of the resistance to aqueous outflow (juxtacanilicular meshwork).

Glaucoma is principally classified into two categories: closed-angle glaucoma and open-angle glaucoma. Closed-angle glaucoma is caused by closure of the anterior angle by contact between the iris and the inner surface of the trabecular meshwork. Closure of this anatomical angle prevents normal drainage of aqueous humor from the anterior chamber of the eye. Open-angle glaucoma is any glaucoma in which the angle of the anterior chamber remains open, but the exit of aqueous through the trabecular meshwork is diminished. The exact cause for diminished filtration is unknown for most cases of open-angle glaucoma. However, there are secondary open-angle glaucomas, which can involve edema or swelling of the trabecular spaces (from steroid use), abnormal pigment dispersion, or diseases such as hyperthyroidism that produce vascular congestion.

Current therapies for glaucoma are directed at decreasing intraocular pressure. This is initially by medical therapy with drops or pills that reduce the production of aqueous humor or increase the outflow of aqueous. However, these various drug therapies for glaucoma are sometimes associated with significant side effects, such as headache, blurred vision, allergic reactions, death from cardiopulmonary complications and potential interactions with other drugs. When the drug therapy fails, surgical therapy is used. Surgical therapy for open-angle glaucoma comprises laser (trabeculoplasty), trabeculectomy, and aqueous shunting implants (after failure of trabeculectomy or if trabeculectomy is unlikely to succeed). Trabeculectomy is a major surgery that is widely used and is augmented with topically applied anticancer drugs such as 5-flurouracil or mitomycin-C to decrease scarring and increase surgical success.

Approximately 100,000 trabeculectomies are performed on Medicare-age patients per year in the United States. This number would increase if the morbidity associated with trabeculectomy could be decreased. The current morbidity associated with trabeculectomy consists of failure (10-15%), infection (a life long risk about 2-5%), choroidal hemorrhage (1%, a severe internal hemorrhage from pressure too low resulting in visual loss), cataract formation, and hypotony maculopathy (potentially reversible visual loss from pressure too low).

If it were possible to bypass the local resistance to outflow of aqueous at the point of the resistance and use existing outflow mechanisms, surgical morbidity would greatly decrease. The reason for this is that the episcleral aqueous veins have a backpressure that would prevent the eye pressure from going too low. This would virtually eliminate the risk of hypotony maculopathy and choroidal hemorrhage. Furthermore, visual recovery would be very rapid and risk of infection would be very small (a reduction from 2-5% to 0.05%). Because of these reasons surgeons have tried for decades to develop a workable surgery for the trabecular meshwork.

The previous techniques that have been tried are goniotomy and trabeculotomy, and other mechanical disruptions of the trabecular meshwork, such as trabeculopuncture, goniophotoablation, laser trabecular ablation and goniocurretage. They are briefly described below.

*Goniotomy/Trabeculotomy*: Goniotomy and trabeculotomy are simple and directed techniques of microsurgical dissection with mechanical disruption of the trabecular meshwork. These initially had early favorable responses in the treatment of open-angle glaucoma. However, long-term review of surgical results showed only limited success in adults. In retrospect, these procedures probably failed secondary to repair mechanisms and a process of "filling in." The filling in is the result of a healing process that has the detrimental effect of collapsing and closing in of the created opening throughout the trabecular meshwork. Once the created openings close, the pressure builds back up and the surgery fails.

*Trabeculopuncture*: Q-switched Neodymium (Nd):YAG lasers also have been investigated as an optically invasive technique for creating full-thickness holes in trabecular meshwork. However, the relatively small hole created by this trabeculopuncture technique exhibits a filling in effect and fails.

*Goniophotoablation/Laser Trabecular Ablation*: Goniophotoablation is disclosed by Berlin in U.S. Pat. No. 4,846,172, and describes the use of an excimer laser to treat glaucoma by ablating the trabecular meshwork. This was not demonstrated by clinical trial to succeed. Hill et al. used an Erbium:YAG laser to create full thickness holes through trabecular meshwork (Hill et al., Lasers in Surgery and Medicine 11:341-346, 1991). This technique was investigated in a primate model and a limited human clinical trial at the University of California, Irvine. Although morbidity was zero in both trials, success rates did not warrant further human trials. Failure again was from filling in of created defects in trabecular meshwork by repair mechanisms. Neither of these is an optimal surgical technique for the treatment of glaucoma.

*Goniocurretage*: This is an ab-interno (from the inside) mechanical disruptive technique. This uses an instrument similar to a cyclodialysis spatula with a microcurrette at the tip. Initial results are similar to trabeculotomy that fails secondary to repair mechanisms and a process of filling in.

Although trabeculectomy is the most commonly performed filtering surgery, *Viscocanulostomy* (VC) and *non-penetrating trabeculectomy* (NPT) are two new variations of filtering surgery. These are ab-externo (from the outside), major ocular procedures in which Schlemm's canal is surgically exposed by making a large and deep scleral flap. In the VC procedure, Schlemm's canal is cannulated and viscoelastic substance injected (which dilates Schlemm's canal and the aqueous collector channels). In the NPT procedure, the inner wall of Schlemm's canal is stripped off after surgically exposing the canal.

Trabeculectomy, VC, and NPT are performed under a conjunctival and scleral flap, such that the aqueous humor is drained onto the surface of the eye or into the tissues located within the lateral wall of the eye. Normal physiological outflows are not used. These surgical operations are major procedures with significant ocular morbidity. When Trabeculectomy, VC, and NPT are thought to have a low chance for success, a number of implantable drainage devices have been used to ensure that the desired filtration and outflow of aqueous humor through the surgical opening will continue. The risk of placing a glaucoma drainage implant also includes hemorrhage, infection, and postoperative double vision that is a complication unique to drainage implants.

Examples of implantable shunts or devices for maintaining an opening for the release of aqueous humor from the anterior chamber of the eye to the sclera or space underneath conjunctiva have been disclosed in U.S. Pat. Nos. 6,007,511 (Prywes), 6,007,510 (Nigam), and 5,397,300 (Baerveldt et al.)

The above embodiments and variations thereof have numerous disadvantages and moderate success rates. They involve substantial trauma to the eye and require great surgical skill by creating a hole over the full thickness of the sclera/cornea into the subconjunctival space. Furthermore, normal physiological outflow pathways are not used. The procedures are mostly performed in an operating room generating a facility fee, anesthesiologist's professional fee and have a prolonged recovery time for vision. The complications of filtration surgery have inspired ophthalmic surgeons to look at other approaches to lowering intraocular pressure.

The trabecular meshwork and juxtacanilicular tissue together provide the majority of resistance to the outflow of aqueous and, as such, are logical targets for surgical removal in the treatment of open-angle glaucoma. In addition, minimal amounts of tissue are altered and existing physiologic outflow pathways are utilized. Trabecular bypass surgery has the potential for much lower risks of choroidal hemorrhage, infection and uses existing physiologic outflow mechanisms. This surgery could be performed under topical anesthesia in a physician's office with rapid visual recovery.

WO-A-01/94784 discloses an implant for the treatment of glaucoma which drains aqueous humor from the anterior chamber. The apparatus comprises a main body having an interior end and a posterior end. A first and second check valve are respectively disposed at the interior and posterior ends. A diaphragm extends across the interior chamber whereby oscillating movement of the diaphragm causes fluid flow through the pump assembly. The diaphragm is powered by a source of electrical power.

Therefore, there is a great clinical need for the treatment of glaucoma by a method that is faster, safer, and less expensive than currently available modalities. Trabecular bypass surgery is an innovative surgery that uses a micro stent, shunt, or other implant to bypass diseased trabecular meshwork alone at the level of trabecular meshwork and use or restore existing outflow pathways. One object of the invention is to provide a means for treating and controlling elevated intraocular pressure in a manner which is simple, affective, and disease site-specific with an implanted micropump and, in some cases, a remote or attached intraocular pressure (IOP) sensor.

Summary of the Invention

Aspects of the invention are defined in the accompanying claims.

In some embodiments, the pump can be located between the inlet and the outlet. In some embodiments, the location outside the anterior chamber can be the Schlemm's canal.

Some embodiments can further include means for powering the pump, such as a power source coupled to the pump. This power source may be mechanical or electrical, for example. The pump may be driven by changes in intraocular pressure that result from at least one of blinking and arterial pulse, both of which cause variations in intraocular pressure.

Some embodiments comprise a sensor that senses intraocular pressure and provides a signal indicative of the sensed intraocular pressure, the pump being responsive to the signal to regulate flow through the pump.

In some embodiments, the sensor can be electrically coupled to the pump. In some embodiments the sensor can be wirelessly coupled to the pump.

In some embodiments, a trabecular pump stent can have inlet portion configured to extend through a portion of the trabecular meshwork of an eye, and an outlet portion configured to extend into Schlemm's canal of the eye, wherein the inlet portion is disposed to the anterior chamber for aqueous communication between the anterior chamber and Schlemm's canal.

In some arrangements, the trabecular pump stent can comprise an inlet portion, configured to extend through a portion of the trabecular meshwork; an outlet portion, configured to extend into Schlemm's canal; and anchoring means for stabilizing the stent in place. The anchoring means may comprise at least one protrusion, configured to anchor through trabecular meshwork into Schlemm's canal.

Some embodiments can comprise an inlet portion configured to extend through a portion of the trabecular meshwork, an outlet portion configured to extend into Schlemm's canal, and means for controlling aqueous flow in one direction. The means for controlling aqueous flow and intraocular pressure may comprise an active method, such as a pump.

Some embodiments of the invention can provide a trabecular pump stent for pumping fluid from an anterior chamber to Schlemm's canal comprising an inlet portion with an inlet terminal exposed to an anterior chamber, an outlet portion with an outlet terminal exposed to Schlemm's canal, and a middle portion having a proximal end and a distal end, wherein a first check valve is located at the proximal end and a second check valve is located at the distal end of the middle portion.

Brief Description of the Drawings

Additional objects and features of the present invention will become apparent from the following Detailed Description of Exemplary Embodiments, when read with reference to the accompanying drawings.

FIG. 1 is a sectional view of an eye.

FIG. 2 is a close-up sectional view, showing the anatomical diagram of trabecular meshwork and the anterior chamber of the eye.

FIGS. 3A-C is an operating schematic for a pressure-pulse driven pump as an implanted trabecular stent.

FIG. 4 is one embodiment of the pressure-pulse driven pump at Schlemm's canal implant location.

FIG. 5 is another embodiment of the pressure-pulse driven pump at anterior-angle implant location.

FIG. 6 depicts an overpressure prevention mechanism.

FIG. 7 depicts an under-pressure protection mechanism.

FIG. 8 is a schematic diagram illustrating a pump and sensor functions for controlling the intraocular pressure of an eye.

FIG. 9 depicts one embodiment of a pressure pulse-driven pump implant.

Detailed Description of Exemplary Embodiments

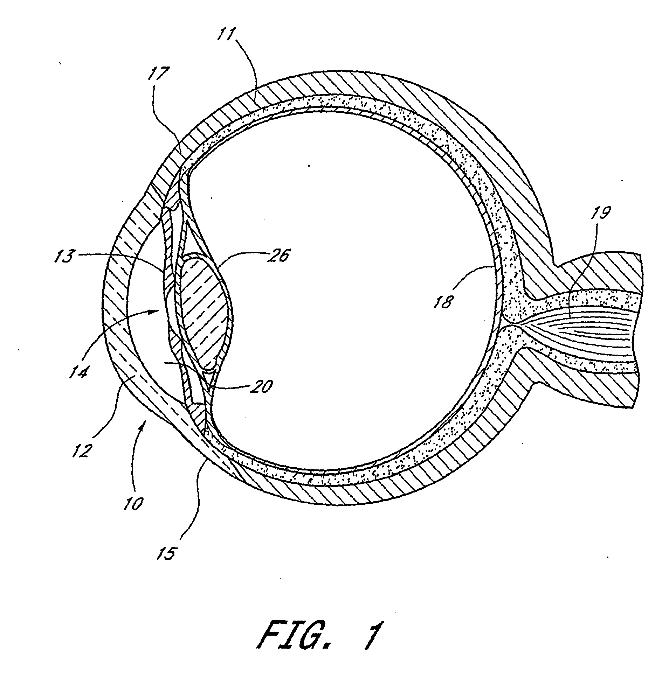

Referring to FIGS. 1 to 9, a trabecular pump is illustrated, which may be attached to or couple with a trabecular stent. In particular, a trabecular stent implant is used to bypass diseased trabecular meshwork having a pump and, in some embodiments, a pressure sensor for controlling the intraocular pressure at a desired level.

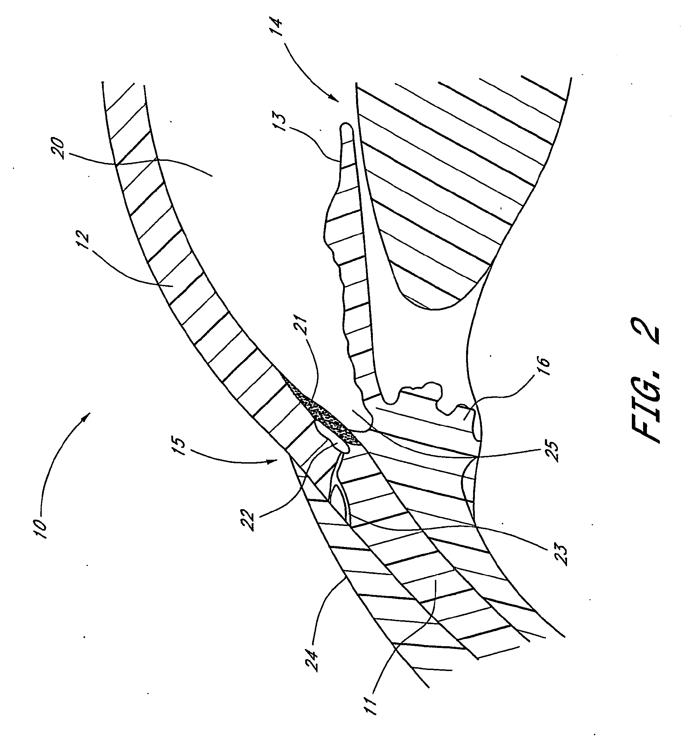

For background illustration purposes, FIG. 1 shows a sectional view of an eye 10, while FIG. 2 shows a close-up view, showing the relative anatomical locations of the trabecular meshwork, the anterior chamber, and Schlemm's canal. Thick collagenous tissue known as sclera 11 covers the entire eye 10 except that portion covered by the cornea 12. The cornea 12 is a thin transparent tissue that focuses and transmits light into the eye and the pupil 14, which is the circular hole in the center of the iris 13 (colored portion of the eye). The cornea 12 merges into the sclera 11 at a juncture referred to as the limbus 15. The ciliary body 16 begins internally in the eye and extends along the interior of the sclera 11 and becomes the choroid 17. The choroid 17 is a vascular layer of the eye underlying retina 18. The optic nerve 19 transmits visual information to the brain and is progressively destroyed by glaucoma.

The anterior chamber 20 of the eye 10, which is bound anteriorly by the cornea 12 and posteriorly by the iris 13 and lens 26, is filled with aqueous. Aqueous is produced primarily by the ciliary body 16 and reaches the anterior chamber angle 25 formed between the iris 13 and the cornea 12 through the pupil 14. In a normal eye, the aqueous is removed through the trabecular meshwork 21. Aqueous passes through trabecular meshwork 21 into Schlemm's canal 22 and through the aqueous veins 23, which merge with blood-carrying veins, and into venous circulation. Intraocular pressure of the eye 10 is maintained by the intricate balance of secretion and outflow of the aqueous in the manner described above. Glaucoma is characterized by the excessive buildup of aqueous fluid in the anterior chamber 20, which produces an increase in intraocular pressure; fluids are relatively incompressible and pressure is directed equally to all areas of the eye.

As shown in FIG. 2, the trabecular meshwork 21 constitutes a small portion of the sclera 11. It is understandable that creating a hole or opening for implanting a device through the tissues of the conjunctiva 24 and sclera 11 is relatively a major surgery as compared to surgery for implanting a device through the trabecular meshwork 21 only.

In a first embodiment, a method for increasing aqueous humor outflow in an eye of a patient to reduce the intraocular pressure therein is described. The method comprises bypassing diseased trabecular meshwork at the level of the trabecular meshwork and thereby restoring existing outflow pathways. Also, a method for increasing aqueous humor outflow in an eye of a patient to reduce an intraocular pressure therein comprises bypassing diseased trabecular meshwork at a level of the trabecular meshwork with a trabecular stent implant and using existing outflow pathways. The trabecular stent implant may be an elongated trabecular stent or other appropriate shape, size or configuration, with a micropump and/or a pressure sensor. In one embodiment of an elongated trabecular stent implant, the trabecular stent has an inlet end, an outlet end, and a lumen therebetween, wherein the inlet end is positioned at an anterior chamber of the eye and the outlet end is positioned at about an exterior surface of the diseased trabecular meshwork. Furthermore, the outlet end may be positioned into fluid collection channels of the existing outflow pathways. Optionally, the existing outflow pathways may comprise Schlemm's canal 22. The outlet end may be further positioned into fluid collection channels up to the level of the aqueous veins, with the trabecular stent inserted either in a retrograde or antegrade fashion with respect to the existing outflow pathways.

In a further embodiment, a method for increasing aqueous humor outflow in an eye of a patient to reduce an intraocular pressure therein comprises (a) creating an opening in trabecular meshwork, wherein the trabecular meshwork comprises an interior side and exterior side; (b) inserting a trabecular pump stent into the opening; (c) activating a micropump on or in the trabecular pump stent; and (d) transporting the aqueous humor by the trabecular pump stent to bypass the trabecular meshwork at the level of the trabecular meshwork from the interior side to the exterior side of the trabecular meshwork.

The trabecular stent implant may comprise a biocompatible material, such as a medical grade silicone, for example, the material sold under the trademark Silastic™, which is available from Dow Coming Corporation of Midland, Michigan, or polyurethane, which is sold under the trademark Pellethane™, which is also available from Dow Corning Corporation. In an alternate embodiment, other biocompatible materials (biomaterials) may be used, such as polyvinyl alcohol, polyvinyl pyrolidone, collagen, heparinized collagen, tetrafluoroethylene, fluorinated polymer, fluorinated elastomer, flexible fused silica, polyolefin, polyester, polysilicon, mixture of biocompatible materials, and the like. In a further alternate embodiment, a composite biocompatible material by surface coating the above-mentioned biomaterial may be used, wherein the coating material may be selected from the group consisting of polytetrafluoroethylene (PTFE), polyimide, hydrogel, heparin, therapeutic drugs, and the like.

It is commonly known that control of intraocular pressure is the primary treatment modality for patients with ocular hypertension or glaucoma. The present invention discloses a pump stent to achieve pressure control at a desired pressure level that is possibly lower than its downstream pressure. The pump stent may comprise a micropump or the like, preferably a valveless or bladeless pump in some embodiments. Alternatively, the pump may comprise a rotary, helical, or propeller blade pump design (not shown), which will be readily known to those of skill in the art. The pump utilizes an energy source to move fluid from the anterior chamber to Schlemm's canal or other physiological outflow areas, for example, collector channels, aqueous veins, episcleral veins, sub-conjunctival spaces or any tissue area adjacent or near the anterior chamber. Many sources of energy are available to drive the pump. By way of example, the energy sources may consist of ocular pressure pulse, blink pressure pulse, solar power, or stored energy (such as batteries). The pump is implanted as a trabecular pump stent or mounted on or around a trabecular stent and utilizes the energy source. A "trabecular pump stent" is herein intended to mean a pump placed within the trabecular meshwork that pumps fluid (for example, aqueous humor) from an anterior chamber to, for example, Schlemm's canal or downstream therefrom.

Implantable Pumps

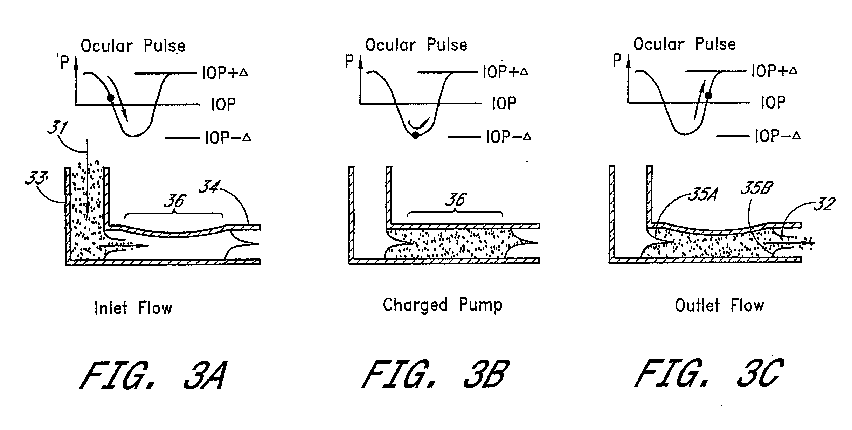

FIGS. 3A-C shows a simple pump with a compressible tube 36, having two check valves 35A, 35B, that is driven by pressure fluctuations in the eye. The energy source for causing the tube to compress may comprise pressure fluctuations, such as ocular pressure pulse, blink pressure pulse, and the like. The energy may be used directly or stored in a battery-type reservoir for future use to drive a compressing unit mounted on the tube. The pump entrance is positioned in or connected to the anterior chamber 20 and the pump exit is positioned in or connected to Schlemm's canal 22 or a point downstream. In one embodiment, the inlet portion 33 and the outlet portion 34 are made of nonexpandable, noncompressible material while the volume of the middle portion 36 can increase and decrease as a result of compression or expansion onto the compressible tube.

In one embodiment, the ocular pressure pulse is used as an example in FIG. 3. The upper part of the figure shows a single cycle in the repetitive ocular pulsations of the intraocular pressure. These are often seen in tomographic pressure tracings with peak-to-peak amplitudes of about 1 to 3 mmHg. The ocular pulse is driven by the heart rate as the blood pressure varies from systole to diastole with each beat of the heart pumping. In the pressure tracings, the mean value is labeled as the IOP (intraocular pressure) of the eye and pressure variations to peak and valley are indicated by the symbol Δ. The black circles on the waveforms represent the cycle points in the operation of the pump. In this embodiment, there are three steps in this pumping process as shown in FIG. 3. In general, the pump comprises an incompressible inlet portion 33, a compressible middle portion 36 located between a first check valve 35A and a second check valve 35B, and an incompressible outlet portion 34. In one embodiment, the inlet portion may be compressible so long as the differential pressure between the inlet portion 33 and the middle portion 36 enables pushing aqueous through the first check valve 35A. In another embodiment, the outlet portion may be compressible so long as the differential pressure between the middle portion 36 and the outlet portion 34 enables pushing aqueous through the second check valve 35B.

In the first step shown in FIG. 3A, the inlet portion 33 of the pump body is filled with aqueous (as shown by arrow 31). When the pressure rises and exceeds the opening pressure of the first check valve 35A, aqueous starts to flow into the middle portion 36 until the pressure equalizes between the inlet portion 33 and the middle portion 36. In the second step shown in FIG. 3B, the tube of the middle portion 36 is compressed by a mechanical pressure or a pumping element using an energy source. The compression onto the tube of the middle portion 36 can be achieved by any conventional means for pinching, wrapping around, or sandwiching with force. When the pressure in the middle portion 36 rises and exceeds the opening pressure of the second check valve 35B, the aqueous is pushed out through the pump exit that is shown by an arrow 32 in FIG. 3C. Further, when the tube of the middle portion 36 has expanded or reversed to its original size, the tube is decompressed and sucks fluid from the inlet portion 33 into the middle portion 36. This pump cycle repeats as the ocular pulse cycle continues. In this way, the pump moves aqueous from the anterior chamber to points downstream in the aqueous outflow system and the check valves prevent reverse flow into the eye. The operating principles of a blink-pressure driven pump is similar, but is actuated at larger pressure differential since blink-induced pressure changes are larger than ocular pulse pressure variations.

Some aspects of the invention provide a pump for pumping fluid from the anterior chamber to Schlemm's canal or downstream therefrom comprises maintaining a pressure at the anterior chamber lower than that at Schlemm's canal or downstream. In the first step, the inlet portion 33 of the pump body is filled with aqueous. Then when the pressure of the middle portion 36 falls below that of the inlet portion 33 so as to open the first check valve 35A, aqueous starts to flow into the middle portion 36 until the pressure equalizes between the inlet portion 33 and the middle portion 36. In a third step, the tube of the middle portion 36 is compressed to push aqueous into the outlet portion 34. The pressure-lowering step of the middle portion can be achieved by any conventional methods, for example, pulling the tube wall radially outwardly using the energy converted from electric or thermoelectric sources (e.g., a battery) mechanism. Alternatively, the material elastic properties of the walls of the middle portion 36 may cause or assist the walls to "spring" back to an uncompressed state. Another method to lower the pressure within the middle portion 36 is by connecting to a suction pump located at a distance away from the pump body.

The pumping volume (ΔV) for each stroke of an implantable pump is dependent on the stroke frequency. The ocular-pulse pump, operating at approximately 72 cycle/minute (heart rate), must pump at a rate that equals the aqueous production rate for the eye (typically 2.4 $\mu$l/min); therefore,

$$\Delta V = (2.4 \ \mu l/min) \ / \ (72 \ cycles/min) = 0.03 \ \mu l/cycle$$

A blink pressure-pulse driven pump operating at approximately 1 cycle/20 seconds must pump aqueous with a stroke volume of:

$$\Delta V = (2.4 \ \mu l/min) \ / \ (3 \ cycles/min) = 0.8 \ \mu l/cycle$$

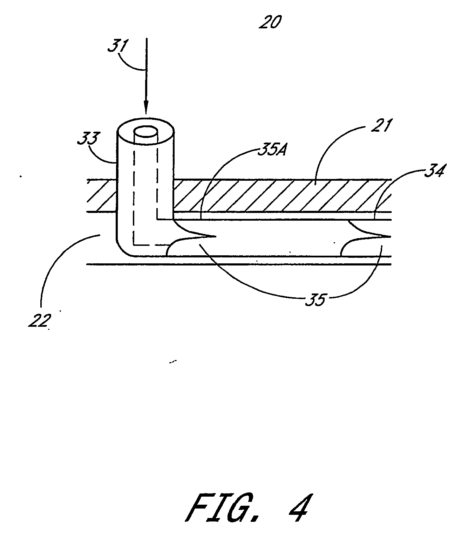

FIG. 4 shows a pressure-pulse driven pump implanted inside Schlemm's canal as a trabecular pump stent of the eye. In one embodiment, pressure pulsations from the anterior chamber press against the trabecular meshwork, which in turn press against the flexible wall of the middle portion 36 of the implanted pump. In another embodiment, pressure pulsations are converted into electricity via a battery mechanism and the electricity is used to drive a mechanical compressing unit for pressing against the flexible tube wall of the middle portion. In some aspects, the pump outlet or exit is located inside Schlemm's canal and aqueous exits the eye through the collector channels and episcleral veins. Other variations include placing, or extending, the exit to the collector channels, aqueous veins, episcleral veins, and sub-conjunctival space. The entrance (as shown by arrow 31) to the pump is located in the anterior chamber 20 of the eye 10.

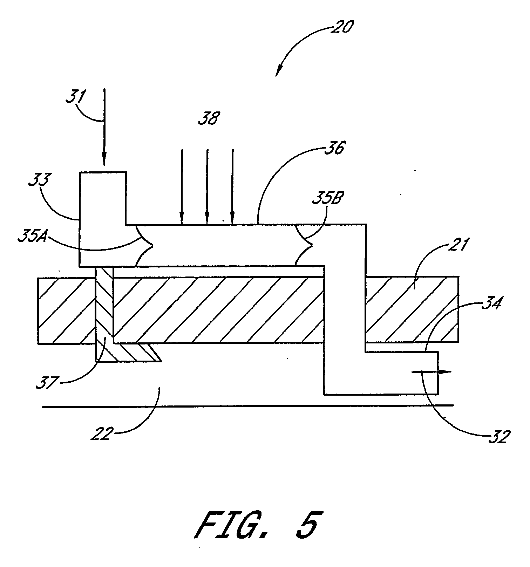

FIG. 5 shows a pressure-pulse driven pump located at an anterior angle implant location. In this example, the pump is anchored into the trabecular meshwork 21 and Schlemm's canal 22 via an anchor 37 and the pump outlet 34. This holds the pump securely against the meshwork in the anterior angle of the eye. Alternatively, anchor points could be in other surrounding tissues and the pump could be placed in other parts of the eye, so long as it is exposed to or coupled to the driving pressure pulse 38. The entrance is in the anterior chamber 20 and the exit is located in Schlemm's canal 22 or any of the various downstream structures. Alternatively, the pump exit could be located in a vein within the iris or eye wall. Rather than anchors, the pump could be held in place by a spring force or other mechanisms such as a circular piece (or part of a circle) in the angle where the extension of the circular-shaped spring pushes the implant against the anterior angle.

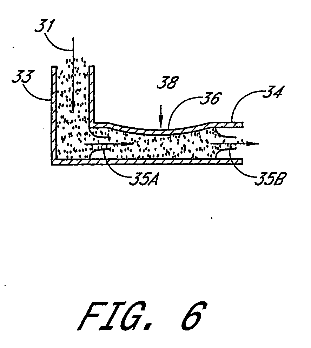

FIG. 6 discloses the overpressure prevention mechanism of a dual check valve pump. If the intraocular pressure exceeds the opening pressures of the valves, then the pump will allow free flow to regulate the intraocular pressure down into the desirable range. In this way, the pump is designed to limit the maximum possible pressure that the eye can achieve. This can be particularly useful during resting periods or periods of reduced cycle frequency where the pump may not be pumping at an adequate rate to keep up with the aqueous production.

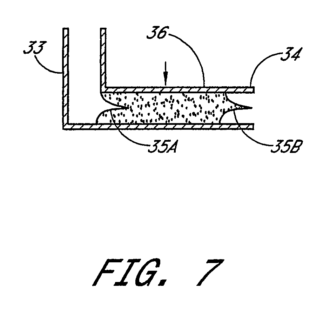

Complementary to the over-pressure protection function, the pump also has a built in under-pressure protection function as shown in FIG. 7. It is desirable not to allow the intraocular pressure to drop below a low threshold, for example, 6 mmHg. Any intraocular pressure below the low threshold is considered hypotonous pressure and is dangerous to the eye since it causes choroidal hemorrhage, choroidal detachment, etc. The pump is self-limiting, since the valves will not open unless the pressure difference across the valve is greater than the opening pressure of the valve. If the maximum intraocular pressure is lower than the threshold pressure, then the valve will not open and aqueous will not leave the eye through the pump exit. The pump will not function until the intraocular pressure rises through the inflow/production of aqueous from the ciliary body.

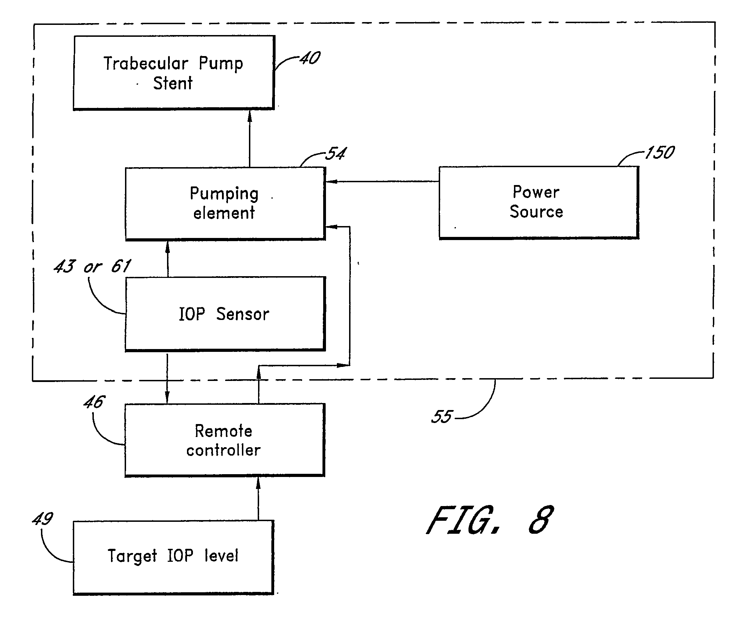

FIG. 8 shows a block diagram illustrating a trabecular pump stent with an IOP sensor for controlling the intraocular pressure of an eye. The block elements within the dashed line 55 are to be placed within the eye in a preferred embodiment. In operation of some embodiments, a target IOP level 49 is prescribed for a patient. The information is logged in with a remote controller 46 and transmitted wirelessly to an implanted pumping element 54 that is a part of the pump stent system. The target IOP level is compared to the sensed IOP data from the IOP sensor 43. The trabecular pump stent will function when the IOP level is lower than sensed IOP.

In one preferred embodiment, to achieve the target IOP level, the trabecular pump 40 with double check valves starts to pump aqueous out of the anterior chamber 20 toward Schlemm's canal 22 or downstream therefrom until the target IOP is reached. The pumping may be accomplished with a mechanical pumping element 54 powered by a power source 150, which may comprise a source of mechanical or electrical energy.

In a preferred embodiment, the target IOP data is transmitted remotely to the pumping element 54. In the meantime, the measured IOP data from the IOP sensor 43 is fed to the pumping element 54 so as to activate the pumping operation whenever the measured IOP is higher than a threshold IOP value.

IOP Sensor and Transmitter

It is one aspect of the present invention to provide a pressure sensor 43 for transmitting a signal either continuously or in response to a remote activation signal from a remote external controller 46. The sensor may comprise energy means for providing power to the sensor, sensing means for determining the pressure and generating a sensing signal indicative thereof and transmitting means for transmitting the IOP data to a remote controller 46. In one embodiment, the transmitter is a radiofrequency transmitter.

An intraocular pressure sensor has been described in, for example, U.S. Patent No. 6,579,235 to Abita et al.

In another aspect, a flashing LED (light emitting diode) may be used to transmit the IOP data to an external controller/display or to the pumping element for pressure control. In one embodiment, the flashing LED is connected to a transducer that converts the IOP data into electrical signal. The LED technology is well known to one ordinary skilled in the art.

In another aspect, a pressure sensor is mounted on a trabecular pump stent for measuring an intraocular pressure and generating a signal indicative of the measured pressure. This signal is then transferred to the pumping element 54.

For continuous monitoring of IOP, a sensor prototype comprises a capacitative-inductive circuit formed from a spiral inductor-diaphragm based capacitor. Upon sensing a change in the IOP level, the pressure-induced displacement of the diaphragm changes the frequency of the circuit. The IOP monitoring is performed telemetrically and does not need to come in contact with the eye. In some embodiments the sensor relies upon an external pickup coil, which can be placed in an unobtrusive device such as spectacles. The prototypes vary from 1.3 mm to 6 mm in diameter, with resolutions of 1.2 to 1.4 mm Hg.

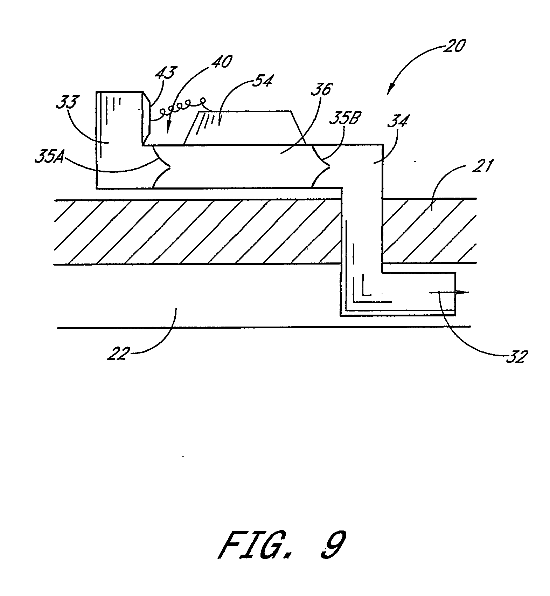

FIG. 9 shows one embodiment of a pressure-pulse driven pump implant with sensing means for providing measured IOP data. The trabecular pump stent 40 comprises an inlet portion 33, an outlet portion 34, and a middle portion 36, bordered by the first check valve 35A and the second check valve 35B. The pump stent 40 further comprises an IOP sensor 43, which feeds the data to a pumping element 54. In one aspect, the pumping element 54 is intimately adhered to or wrapped around the wall of the middle portion 36 and has the capability of providing suction enabling the tube wall to expand and providing pressure enabling the tube wall to compress. The pumping element 54 can be powered by mechanical energy or electricity derived from various energy sources, including the conversion of mechanical to electrical energy.

Some aspects of the invention provide an intraocular pumping system, comprising setting a target IOP level, sensing the real-time IOP and comparing to the target level, and pumping aqueous out of the anterior chamber once the sensed IOP is higher than the target IOP.

From the foregoing description, it should be appreciated that a novel approach for sensing and controlling the IOP at a target level has been disclosed for regulating intraocular pressure. While the invention has been described with reference to specific embodiments, the description is merely illustrative and is not to be construed as limiting the invention. Various modifications and applications may occur to those who are skilled in the art without departing from the scope of the invention, as described by the appended claims and their equivalents.

## Claims

1. An apparatus for transporting aqueous humor from the anterior chamber of an eye, the apparatus comprising:

   an inlet configured to receive aqueous humor from the anterior chamber,
   an outlet configured to ouput aqueous humor to a physiologic outflow pathway located outside the anterior chamber, and
   a pump configured to pump aqueous humor from the inlet to the outlet, the pump comprising a pair of substantially one-way valves that are spaced to provide a fluid chamber therebetween, said fluid chamber being configured such that a fluid volume within said fluid chamber changes in response to a variation in intraocular pressure, to drive the pump.

2. The apparatus of Claim 1, wherein the physiologic outflow pathway located outside the anterior chamber is Schlemm's canal.

3. The apparatus of Claim 1 or Claim 2, further comprising a power source coupled to the pump.

4. The apparatus of Claim 3, wherein said power source is mechanical.

5. The apparatus of any preceding claim, wherein said pump is configured to be driven at least partially by changes in intraocular pressure that result from at least one of blinking and arterial pulses.

6. The apparatus of any preceding claim, further comprising:

   a sensor configured to sense intraocular pressure and provide a signal indicative of the sensed intraocular pressure, wherein said pump is responsive to said signal to regulate flow through the pump.

7. The apparatus of Claim 6, wherein said sensor is electrically coupled to said pump.

## Patentansprüche

1. Vorrichtung zum Transportieren von wäßrigem Humor von der Vorderkammer eines Auges, wobei die Vorrichtung umfaßt:

einen Einlaß, der dafür ausgestaltet ist, wäßrigen Humor von der Vorderkammer aufzunehmen,
einen Auslaß, der dafür ausgestaltet ist, wäßrigen Humor in einen außerhalb der Vorderkammer liegenden physiologischen Abflußpfad abzugeben, und
eine Pumpe, die dafür ausgestaltet ist, wäßrigen Humor von dem Einlaß zu dem Auslaß zu pumpen, wobei die Pumpe ein Paar von Ventilen umfaßt, die im wesentlichen Ein-Weg-Ventile sind und zueinander beabstandet sind, um eine Fluidkammer dazwischen bereitzustellen, wobei die Fluidkammer so ausgestaltet ist, daß ein Fluidvolumen innerhalb der Fluidkammer sich in Reaktion auf eine Änderung des Augeninnendrucks verändert, um die Pumpe anzutreiben.

2. Vorrichtung nach Anspruch 1, wobei der außerhalb der Vorderkammer liegende physiologische Abflußpfad ein Schlemm'scher Kanal ist.

3. Vorrichtung nach Anspruch 1 oder Anspruch 2, welche weiterhin eine mit der Pumpe gekoppelte Energiequelle umfaßt.

4. Vorrichtung nach Anspruch 3, wobei die Energiequelle mechanisch ist.

5. Vorrichtung nach einem der vorangegangenen Ansprüche, wobei die Pumpe so ausgestaltet ist, daß sie wenigstens teilweise durch Veränderungen des Augeninnendrucks, die aus wenigstens entweder Blinzeln oder arteriellen Pulsen resultieren, betrieben wird.

6. Vorrichtung nach einem der vorangegangenen Ansprüche, welche weiterhin folgendes umfaßt:

einen Sensor, der dafür ausgestaltet ist, den Augeninnendruck zu erfassen und ein Signal bereitzustellen, welches den erfaßten Augeninnendruck anzeigt, wobei die Pumpe auf das Signal so reagiert, daß der Fluß durch die Pumpe reguliert wird.

7. Vorrichtung nach Anspruch 6, wobei der Sensor elektrisch mit der Pumpe gekoppelt ist.


**Revendications**

1. Appareil pour transporter de l'humeur aqueuse depuis la chambre antérieure d'un oeil, l'appareil comportant :

une entrée configurée pour recevoir de l'humeur aqueuse depuis la chambre antérieure;
une sortie configurée pour débiter de l'humeur aqueuse vers un circuit d'écoulement physiologique placé à l'extérieur de la chambre antérieure ; et
une pompe configurée pour pomper de l'humeur aqueuse de l'entrée vers la sortie, la pompe comportant une paire de clapets sensiblement unidirectionnels qui sont espacés pour former entre eux une chambre à fluide, ladite chambre à fluide étant configurée de façon qu'un volume de fluide dans ladite chambre à fluide varie en réponse à une variation de la pression intraoculaire, pour entraîner la pompe.

2. Appareil selon la revendication 1, dans lequel le circuit d'écoulement physiologique placé à l'extérieur de la chambre antérieure est le canal de Schlemm.

3. Appareil selon la revendication 1 ou la revendication 2, comportant en outre une source d'énergie reliée à la pompe.

4. Appareil selon la revendication 3, dans lequel ladite source d'énergie est mécanique.

5. Appareil selon l'une quelconque des revendications précédentes, dans lequel ladite pompe est configurée pour être entraînée au moins partiellement par des variations de la pression intraoculaire qui résultent d'au moins l'un d'un clignement et de pulsations artérielles.

6. Appareil selon l'une quelconque des revendications précédentes, comportant en outre :

un capteur configuré pour capter la pression intraoculaire et produire un signal représentatif de la pression intraoculaire captée, dans lequel ladite pompe réagit audit signal pour réguler l'écoulement dans la pompe.

7. Appareil selon la revendication 6, dans lequel ledit capteur est relié électriquement à ladite pompe.

**FIG. 1**

EP 1 545 655 B1

*FIG. 2*

Ocular Pulse

P

——— IOP+△

——— IOP

——— IOP−△

*31*

*33*

*36*

*34*

Inlet Flow

## FIG. 3A

Ocular Pulse

P

——— IOP+△

——— IOP

——— IOP−△

*36*

Charged Pump

## FIG. 3B

Ocular Pulse

P

——— IOP+△

——— IOP

——— IOP−△

*35A*  *35B*  *32*

Outlet Flow

## FIG. 3C

FIG. 4

EP 1 545 655 B1

FIG. 5

FIG. 6

FIG. 7

EP 1 545 655 B1

*FIG. 8*

FIG. 9

EP 1 545 655 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4846172 A
- US 6007511 A, Prywes
- US 6007510 A, Nigam
- US 5397300 A, Baerveldt
- WO 0194784 A
- US 6579235 B, Abita

**Non-patent literature cited in the description**

- **HILL et al.** *Lasers in Surgery and Medicine,* 1991, vol. 11, 341-346